# EUROPEAN PATENT SPECIFICATION

(11) **EP 4 121 156 B1**
(45) Date of publication and mention of the grant of the patent: **26.02.2025**
(21) Application number: 21715706.4
(22) Date of filing: 08.03.2021
(51) Int. Cl.: A61M 25/01, A61M 25/09

(54) **MEDICAL DEVICES WITH DEFLECTIVE DISTAL ENDS**
MEDIZINISCHE VORRICHTUNGEN MIT DEFLEKTIVEN DISTALEN ENDEN
DISPOSITIFS MÉDICAUX À EXTRÉMITÉS DISTALES DÉFLECTRICES

(30) Priority: 16.03.2020 US 202016820616
(43) Date of publication of application: 25.01.2023
(73) Proprietor: Stryker Corporation, Portage, MI 49002-9711 (US); Stryker European Operations Limited, Carrigtwohill, Co. Cork T45 HX08 (IE)
(72) Inventor: LAYMAN, Ted W., Park City, Utah 84098 (US); NORTHROP, Clay W., Salt Lake City, Utah 84109 (US); GLITHERO, Jason Iain, Oakland, California 94602 (US)
(74) Representative: Hauck Patentanwaltspartnerschaft mbB
(86) International application number: PCT/US2021/021313
(87) International publication number: WO 2021/188314

(56) References cited:
- JP-A- H08 141 971
- US-A- 4 944 727
- US-A- 5 019 040
- US-A- 5 025 799
- US-A- 5 230 348
- US-A1- 2005 283 095
- US-A1- 2019 374 746

## Description

### FIELD

The field of the application relates to medical devices, and more specifically, to deflective guidewires, pushwires, and delivery wires for medical devices, and medical devices having such guidewires, pushwires, and delivery wires.

### BACKGROUND

The use of intravascular implants, such as stents, stent grafts, flow-diverters, aneurysm occlusive devices, vena cava filters, etc., has become an effective method for treating many types of vascular disease. In general, a suitable intravascular implantable device is inserted into the vascular system of the patient and navigated through the vasculature to a targeted implantation site using a delivery system.

Minimally invasive delivery systems include catheters, push or delivery wires, and the like, are percutaneously introduced into the patient's vasculature over a guidewire. Commonly used vascular application to access a target site in a patient involves inserting a guidewire through an incision in the femoral artery near the groin, and advancing the guidewire until it reaches the target site. Then, a catheter is advanced over the guidewire until an open distal end of the catheter is disposed at the target site. Simultaneously or after placement of the distal end of the catheter at the target site, an intravascular implant is advanced through the catheter via a push or delivery wire.

In certain applications, such as neurovascular, the guidewires, pushwires, and delivery wires are required to navigate tortuous and intricate vasculature, including travel within relatively fragile blood vessels in the brain, and are often required to change direction and to even double back on themselves. Thus, these wires (i.e., guidewires, pushwires, and delivery wires) should have suitable flexibility, kink resistance, pushability and torqueability to successfully navigate the vasculatures, such as cerebral and peripheral vasculature. Suitable flexibility and kink resistance of these wires allow them to navigate through a relatively tight bend without breaking or permanently deforming. Further, the forces applied at the proximal end of these wires should be transferred to the distal ends for suitable pushability (axial rigidity) and torqueability (rotation). Achieving a balance between these features is highly desirable. For example, the guidewires, push and/or delivery wires may comprise variable stiffness sections (e.g., varying ratio of material, including selective reinforcement, such as braids, coils, or the like) suitable to provide sufficient flexibility, kink resistance, pushability, and torqueability to allow navigation through vasculature.

Further, in certain applications, it may be desirable for the distal end of guidewires, pushwires, and/or delivery wires to be configured to deflect or bend during navigation through blood vessels, and/or when near a target site in the vasculature, which allows them to access the target site. Some guidewires, pushwires, and/or delivery wires have a pre-bent distal end to reach particular tight bends in the vasculature. However, these pre-bent wires may end up inadvertently colliding into, catching and/or scraping the inner wall of the vessel, especially in a tortuous and intricate vascular system, and at bifurcated vessels walls, aneurysms, and other anatomical features, during navigation and advancement of the wires. Such navigational difficulties may undesirably increase the time needed for performing a medical procedure, and may further increase the risk of trauma or damage to the blood vessels.

US 4944727 A describes a maneuverable distal apparatus including a temperature-activated memory element moving in a first direction to assume a predetermined shape when heated to a predetermined temperature and control means for selectively heating the memory element so that the memory element is moved in the first direction. A spring is provided for yieldably urging the memory element in a second direction away from the first direction upon cooling of the memory element to a temperature less than the predetermined temperature so that the memory element is moved to assume a shape other than the predetermined shape.

US 2019374746 A1 describes a steerable surgical device including a flexible joint positioned between first and second tubular elements, with multiple shape memory alloy wire elements extending across or through the joint being circumferentially spaced relative to one another and independently actuatable to effectuate pivotal movement between the first and second tubular elements to provide enhanced maneuverability relative to single degree of freedom steerable devices.

US 5025799 A describes a medical guide wire or the like that is comprised in part or entirely of one or more heat activated memory alloys alone or in conjunction with one or more non-heat activated memory materials. The tip or any portion of a guide wire comprised of memory alloy or components thereof can be aimed, deflected or steered on command by applying heat to the alloy.

US 20050283095 A1 describes an actuating medical device and methods for making and using the same. The actuating medical device may include a proximal shaft portion having a distal end region, an actuating shaft portion attached to the distal end region, one or more actuating members coupled to or otherwise disposed adjacent the actuating shaft portion, and a distal shaft portion attached to the actuating shaft portion. The actuating shaft portion may include a shape memory material and may be adapted to shift between a first configuration and a second configuration. Using the actuating medical device may include positioning the actuating medical device in a blood vessel and shifting the actuating shaft portion between the first and second configurations.

### SUMMARY

The invention is based on the task of providing an improved medical device of the type described above. The task is solved by a device according to claim 1. Advantageous embodiments are indicated in the dependent claims.

The invention is defined by a medical device according to claim 1.

According to an example that may, if applicable, provide details to further specify embodiments claimed or described in this application a medical device includes: an elongated member having a proximal end, a distal end, and a body extending between the proximal end and the distal end; wherein at least a first portion of the elongated member comprises a first segment made from a shape-memory material, and a second segment made from a non-shape-memory material, the first portion being a distal portion of the elongated member; wherein the first segment and the second segment of the distal portion of the elongated member are secured to each other along their respective longitudinal sides; and wherein the first segment is configured to undergo length change to cause the distal portion of the elongated member to bend.

Optionally, the first segment is configured to change length in response to a temperature that is above a body temperature.

Optionally, the temperature is at least ten degrees Fahrenheit (5,55°C) above the body temperature.

Optionally, the medical device further includes an energy source coupled to the elongated member, wherein the energy source is configured to deliver a current to the elongated member to increase a temperature of the distal portion of the elongated member.

Optionally, the medical device further includes a user interface configured to allow a user to adjust the current from the energy source to affect a corresponding change in a curvature of a bending of the distal portion of the elongated member.

Optionally, the shape-memory material of the first segment comprises shape-memory Nitinol.

Optionally, the non-shape-memory material of the second segment comprises non-shape-memory Nitinol.

Optionally, the elongated member comprises a second portion proximal to the distal portion.

Optionally, the second portion and the distal portion of the elongated member are made from different materials.

Optionally, the second portion comprises stainless steel, and the distal portion comprises Nitinol.

Optionally, the second portion of the elongated member comprises non-shape-memory Nitinol, and the first segment of the distal portion of the elongated member comprises shape-memory Nitinol.

Optionally, the medical device further includes a coil coupled to the elongated member.

Optionally, the medical device further includes a jacket or a slotted tube disposed around at least the distal portion of the elongated member.

Optionally, the medical device further includes a marker coupled to the distal portion of the elongated member.

A medical device includes: an elongated member having a proximal end, a distal end, and a body extending between the proximal end and the distal end; wherein at least a first portion of the elongated member comprises a first segment and a second segment, the first portion being a distal portion of the elongated member; wherein the first segment and the second segment of the distal portion of the elongated member are secured to each other along their respective longitudinal sides; and wherein the first segment is configured to undergo length change in response to a temperature that is above a body temperature, and wherein the second segment is configured to undergo zero length change or less length change compared to the first segment in response to the temperature.

Optionally, the temperature is at least ten degrees Fahrenheit (5,55°C) above the body temperature.

Optionally, the first segment is configured to undergo the length change to cause the distal portion of the elongated member to bend.

Optionally, the medical device further includes an energy source coupled to the elongated member, wherein the energy source is configured to deliver a current to the elongated member to increase a temperature of the distal portion of the elongated member.

Optionally, the medical device further includes a user interface configured to allow a user to adjust the current from the energy source to affect a corresponding change in a curvature of a bending of the distal portion of the elongated member.

Optionally, the first segment comprises a shape-memory material, and the second segment comprises a non-shape-memory material.

Optionally, the shape-memory material of the first segment comprises shape-memory Nitinol, and wherein the non-shape-memory material of the second segment comprises non-shape-memory Nitinol.

Optionally, the elongated member comprises a second portion proximal to the distal portion.

Optionally, the second portion and the distal portion of the elongated member are made from different materials.

Optionally, the second portion comprises stainless steel, and the distal portion comprises Nitinol.

Optionally, the second portion of the elongated member comprises non-shape-memory Nitinol, and the first segment of the distal portion of the elongated member comprises shape-memory Nitinol.

Optionally, the medical device further includes a coil coupled to the elongated member.

Optionally, the medical device further includes a jacket or a slotted tube disposed around at least the distal portion of the elongated member.

Optionally, the medical device further includes a marker coupled to the distal portion of the elongated member.

Other and further aspects and features will be evident from reading the following detailed description.

### BRIEF DESCRIPTION OF THE DRAWINGS

The drawings illustrate the design and utility of embodiments, in which similar elements are referred to by common reference numerals. These drawings are not necessarily drawn to scale. In order to better appreciate how the above-recited and other advantages and objects are obtained, a more particular description of the embodiments will be rendered, which are illustrated in the accompanying drawings. These drawings depict only exemplary embodiments and are not therefore to be considered limiting in the scope of the claims.
**FIGS. 1A-1B** are cross-sectional views of a guidewire being introduced into a bifurcated vasculature;
**FIGS. 2A-2B** are cross-sectional views of another guidewire being introduced into a bifurcated vasculature;
**FIG. 3** illustrates a medical device having a catheter for delivering an implant;
**FIGS. 4A-4B** illustrate an example of a guidewire, particularly showing a distal segment of the guidewire;
**FIGS. 5-6** are cross-sectional views of the guidewire of **FIGS. 4A-4B****,** particularly showing a distal end portion of the guidewire being introduced into a bifurcated vasculature;
**FIGS. 7A-9** are cross-sectional views of different embodiments of medical devices.

### DETAILED DESCRIPTION

Various embodiments are described hereinafter with reference to the figures. The figures are not necessarily drawn to scale, and elements of similar structures or functions are represented by like reference numerals throughout the figures. It should also be understood that the figures are only intended to facilitate the description of the embodiments, and are not intended as an exhaustive description of the claimed inventions, or as a limitation on the scope thereof, which is defined only by the appended claims.

In addition, the respective illustrated embodiments need not have all of the depicted features. Also, an aspect or an advantage described in conjunction with a particular embodiment is not necessarily limited to that embodiment and can be practiced in any other embodiments even if not so illustrated, or if not so explicitly described.

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

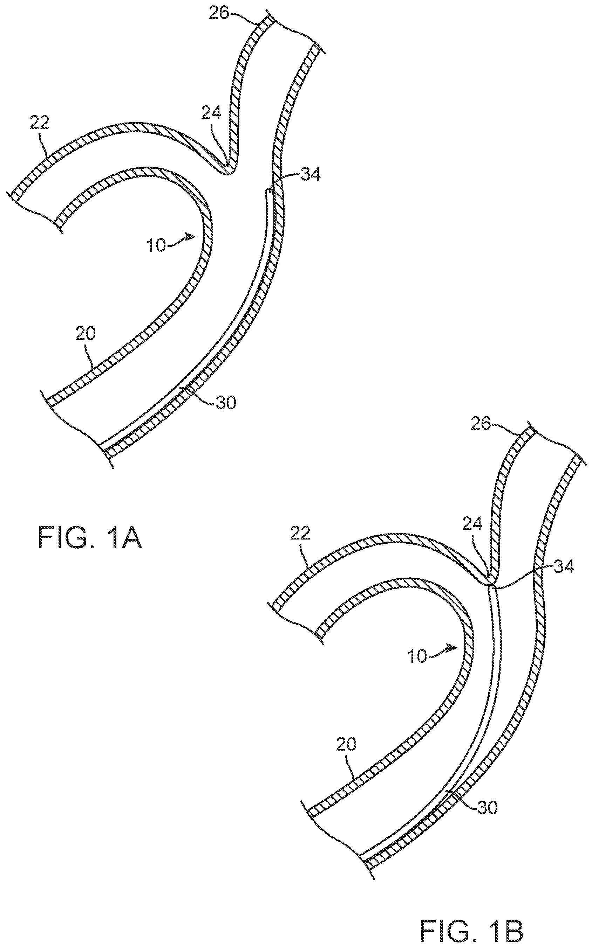

**FIGS. 1A-1B** illustrate a method of accessing a bifurcated vasculature 10 using a guidewire 30 having a distal end 34. A distal segment (including the distal end 34) of the guidewire 30 is composed of a single material (e.g., Nitinol) with shape-memory properties, which can be thermally or electrically activated. The bifurcated vasculature 10 includes a main blood vessel 20, a first blood vessel branch 22, a second blood vessel branch 26, and a bifurcated angle 24 between the first branch 22 and the second 26 branch. The guidewire 30 is advanced through the main blood vessel 20 and maneuvered to access a target site within the first blood vessel branch 22. The guidewire 30 advances along a path of least resistance by sliding through the main blood vessel 20, favoring access to the second blood vessel branch 26 **(****FIG. 1A****).** While corrective action may be taken by the attending physician to maneuver the guidewire 30 into the desired first blood vessel branch 22, in some cases, the distal end 34 of the guidewire 30 may still catch and bump against the bifurcated angle 24 **(****FIG. 1B****).** This is because single material guidewire may suffer from poor shape-retention when its distal segment is deflected, due to the extreme ductility of Nitinol when operating below its active temperature (e.g., Austenite finish temperature). The bumping against the bifurcated angle 24 by the distal end 34 of the guidewire 30 can damage the blood vessel, particularly, a relatively fragile neurovascular vessel. This may also increase the time of the medical procedure when several attempts to maneuver the guidewire 30 towards the desired first blood vessel branch 22 are unsuccessful.

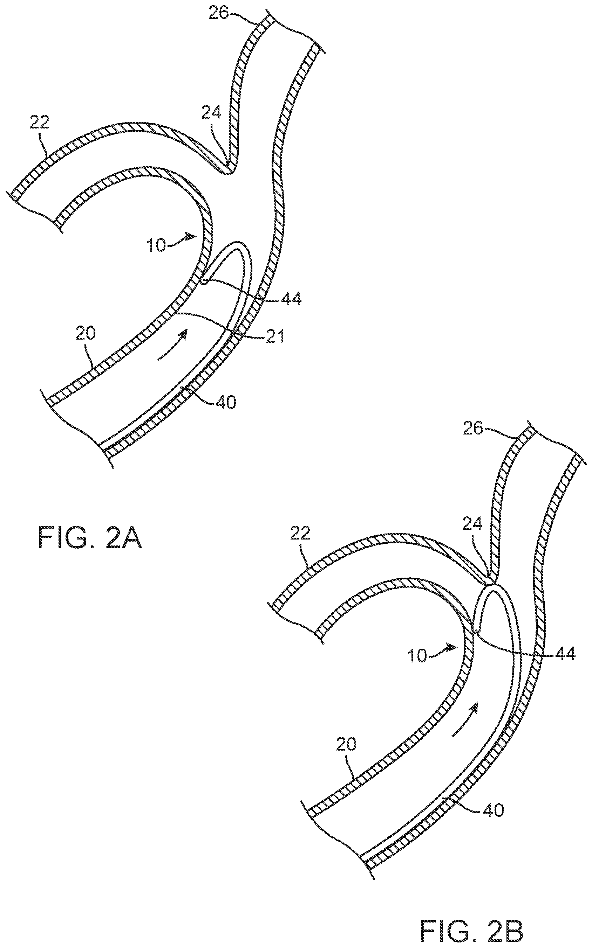

In some cases, a guidewire may have a pre-bent distal segment to assist navigation through certain blood vessels. However, such guidewire may unintentionally cause trauma to the blood vessel. By way of further illustration, **FIGS. 2A-2B** illustrate a method of accessing the bifurcated vasculature 10, which involves use of a guidewire 40 having a pre-bent distal segment 44. When the guidewire 40 is navigated towards the desired first blood vessel branch 22, the guidewire 40 may favor access to the unintended second blood vessel branch 26 due to the geometry of the bifurcated vasculature 10. As a result, the pre-bent distal segment 44 of the guidewire 40 may catch and cause trauma to the inner walls 21 of the blood vessel 20 **(****FIG. 2A****),** and/or may catch and cause trauma to the bifurcated angle 24 **(****FIG. 2B****).** As shown in **FIG. 2B****,** after the pre-bent distal segment 44 abuts against the bifurcated angle 24, further attempts to advance the distal segment 44 distally may deflect the distal segment 44 towards the proximal end, and may further increase the risk of trauma to the blood vessel.

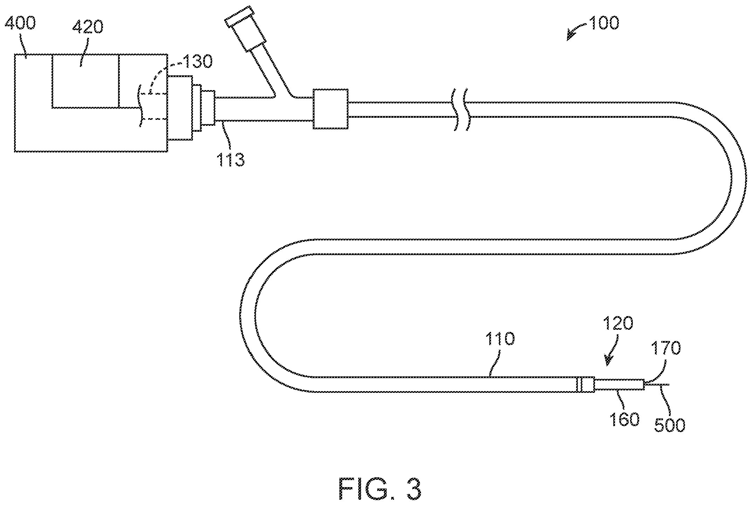

**FIG. 3** illustrates an implant delivery system 100 in accordance with some embodiments. The implant delivery system 100 comprises an elongated sheath 110, an elongated tubular member 120 slidably disposed in the elongated sheath 110, and a guidewire (or wire) 500 slidably disposed in the elongate sheath 110. In some embodiments, the guidewire 500 is configured to access a blood vessel in a patient. In other embodiments, the guidewire 500 may be configured to deliver an implant (not shown). In such cases, the guidewire 500 may function as a delivery wire or a pushwire.

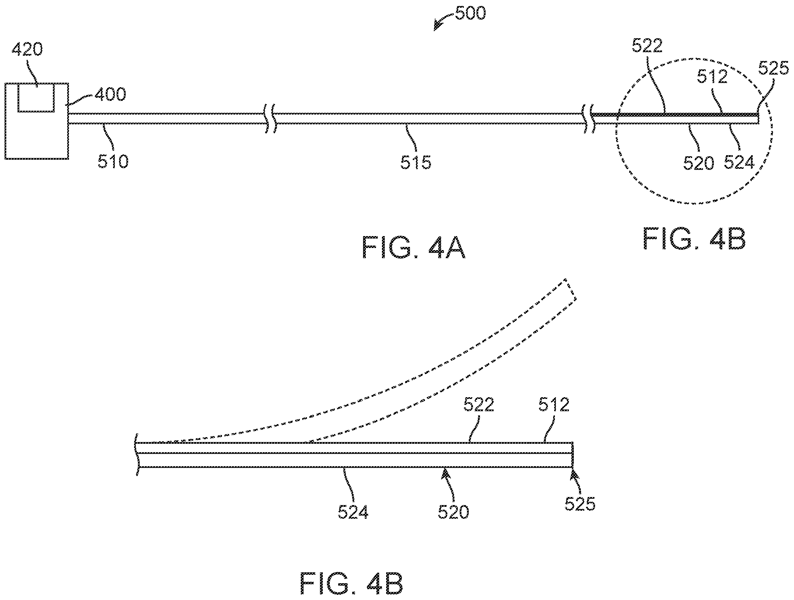

The elongated member 120 has a tubular configuration, and may, e.g., take the form of a sheath, catheter, micro-catheter or the like. The elongated member 120 has a proximal end 130, a distal end 160, and a lumen 170 extending through the elongated member 120 between the proximal end 130 and the distal end 160. The proximal end 130 of the elongated member 120 remains outside of the patient and accessible to the operator when the implant delivery system 100 is in use, while the distal end 160 of the elongated member 120 is sized and dimensioned to reach remote locations of a vasculature. The elongated member 120 is advanced over a guidewire 500 (an example of which will be described with reference to **FIGS. 4A-4B****)** until the distal end 160 of the elongated member 120 is disposed at a target site. Simultaneously or after placement of the distal end 160 of the elongated member 120 at the target site, an intravascular implant may be advanced through the elongated member 120 via the guidewire 500.

As shown in **FIG. 3****,** the implant delivery system 100 also includes a handle 400 coupled to the proximal end of the guidewire 500 **(****FIG. 4A****).** The handle 400 of the implant delivery system 100 includes a user interface 420 configured for allowing a user (e.g., physician, technician or the like) to control a bending of the guidewire 500. The user interface 420 is illustrated as being implemented at the handle 400, but in other embodiments, the user interface 420 may be implemented as another device that is separate from the handle 400. For example, in other embodiments, the user interface 420 may be a computer or any electronic device (e.g., cell phone, tablet, etc.) that is capable of generating electrical signals and/or radiofrequency signals. The user interface 420 may include an electrical controller, power supply or the like, configured to deliver current to the components of the implant delivery system 100 (e.g., to the guidewire 500). In some embodiments, the user interface 420 may include one or more controls, which may be one or more physical button(s), knob(s), switch(es), etc. In other embodiments, the one or more controls may be a touch screen with graphical elements configured to allow the user to selectively activate the guidewire 500 for bending the guidewire 500.

In some embodiments, the handle 400 may also be optionally coupled to the proximal end 130 of the elongated member 120, and/or to a proximal end 113 of the elongated sheath 110. In such cases, the user interface 420 may also allow a user to control a bending of the elongated member 120, and/or the elongated sheath 110. The elongated member 120 and/or the elongated sheath 110 may include actuation elements (e.g., steering wires), which are actuatable in response to tension forces provided by the user interface 420, to thereby bend the elongated member 120 and/or elongated sheath 110. The user interface 420 may include one or more controls for allowing the user to apply tension to the steering wires. In some embodiments, the one or more controls may be one or more physical button(s), knob(s), switch(es), etc. In other embodiments, the one or more controls may be a touch screen with graphical elements configured to allow the user to activate the actuating element(s) of the elongated member 120 and/or elongated sheath 110.

In other embodiments, the elongated member 120 and/or the elongated sheath 110 may not include any steering wires. In such cases, the bending of the elongated member 120 and/or the elongated sheath 110 may be controlled by the guidewire 500.

In further embodiments, the delivery system 100 may not include the sheath 110 and/or the elongated member 120.

Furthermore, in other embodiments, the system 100 may not be an implant delivery system. Instead, the system 100 may be other types of medical devices, or components of other types of medical devices. For example, the system 100 with the guidewire 500 may be a part of a drug delivery system, a biopsy system, a treatment system that includes an energy source, etc.

**FIGS. 4A-4B** illustrate the guidewire 500 in accordance with some embodiments. As shown in the figures, the guidewire 500 includes a proximal end 510, a distal end 512, and a body 515 extending between the proximal end 510 and the distal end 512. The guidewire 500 also includes a distal portion 520 having the distal end 512. The guidewire 500 has a linear configuration that is relatively straight (compared to a bent configuration) at room and/or body temperature, yet flexible to bend when subjected to external forces. The guidewire 500 further includes variable stiffness sections from higher stiffness at a proximal portion, while gradually reducing stiffness along the body 515, to a lower stiffness along the distal portion 520. Such configuration provides sufficient flexibility, kink resistance, pushability, and torqueability for the guidewire 500 for navigation through vasculature. Alternatively, the variable stiffness sections of the guidewire 500 may be distinct instead of gradual. For example a first portion of the guidewire 500 that is closer to the proximal end 510 than to the distal end 512 may have a first stiffness, a second portion of the guidewire 500 that is distal to the second portion may have a second stiffness, and a third portion of the guidewire 500 that is distal to the second portion may have a third stiffness. The third portion may be the distal portion 520. The first stiffness may be higher than the second stiffness, and the second stiffness may be higher than the first stiffness.

It should be noted that the term "body temperature", as used in this specification, may refer to a range of temperatures, such as a temperature range of 95° to 107° Fahrenheit (35°C to 41,6667°C), or more preferably a temperature range of 96° to 100° Fahrenheit (35.5556°C to 37.7778°C), or more preferably a temperature range of 97° to 99° Fahrenheit (36.1111°C to 37.2222°C). Also, as used in this specification, the term "room temperature" may refer to any temperature that is different from the body temperature. For example, room temperature may be any temperature that is lower than body temperature. In some embodiments, the room temperature may be any temperature that is at least 10° Fahrenheit (5,55°C) below the body temperature, or that is at least 20° Fahrenheit (11.11°C) below the body temperature.

As shown in **FIGS. 4A-4B****,** the distal portion 520 of the guidewire 500 comprises a first segment 522 and a second segment 524, wherein the first segment 522 is coupled to the second segment 524. The distal portion 520 of the guidewire 500 may optionally also include a radio-opaque marker 525. The first segment 522 of the distal portion 520 of the guidewire 500 is configured to change to a more curvilinear configuration from its relatively straight configuration in response to temperature change(s), while the second segment 524 is independent of temperature changes. When the first segment 522 changes its shape from its relatively straight configuration in response to temperature change(s), the first segment 522 displaces or moves the second segment 524 along with it, so that the distal end 512 of the distal portion 520 of the guidewire 500 deflects, as represented by the broken lines in **FIG. 4B****,** which will be described in further details below. In the illustrated embodiments, the first segment 522 is configured to contract in response to temperature change(s). In such cases, a contraction of the first segment 522 will cause the distal portion 520 of the guidewire 500 to bend in a direction that is towards the side of the first segment 522. In accordance with the invention, the first segment 522 is configured to extend or elongate in response to temperature change(s). In such cases, an extension of the first segment 522 will cause the distal portion 520 of the guidewire 500 to bend in a direction that is towards the side of the second segment 524.

In some embodiments, the first segment 522 of the distal portion 520 of the guidewire 500 is composed of shape-memory Nitinol, and the second segment 524 of the distal portion 520 of the guidewire 500 is composed non-shape-memory Nitinol. In other embodiments, the first segment 522 may be made from other shape-memory materials, such as shape-memory metal, shape-memory alloy, etc. Also, in other embodiments, the second segment 524 may be made from other non-shape-memory materials, such as non-shape-memory metal, non-shape memory alloy, etc. In accordance with the invention, the first segment 522 and second segment 524 of the distal portion 520 are fixedly attached (e.g., laminated) at one or more points along a longitudinal axis of the guidewire 500 by suitable techniques, such as solder, adhesive, laser spot welds, or their like.

In some embodiments, the first segment 522 of the distal portion 520 is configured to be thermo-electrically actuated to deflect the distal portion 520 of the guidewire 500. In the illustrate embodiments, the first segment 522 has been thermo-mechanically processed so that it will shorten (e.g., contracts) when heated above an activation temperature. The second segment 524 does not include a shape memory behavior, and thus, when the distal portion 520 of the guidewire 500 is heated above the activation temperature, the first segment 522 shortens while the second segment 524 retains its length. The shortening of the first segment 522 relative to the second segment 524 will cause the distal portion 520 of the guidewire 500 to bend. The shortening/contraction of the first segment 522 creates a deflection of the distal portion 520 of the guidewire 500, as represented by the broken lines in **FIG. 4B****.**

In some embodiments, the activation temperature may be an Austenite finish temperature (AF). The term Austenite finish temperature ("Af"), as used in this specification, is the temperature at which martensite to austenite transformation is completed on heating of a material, such as metal alloy (e.g., Nitinol). When the material is fully martensite and is subjected to heating, austenite starts to form at the austenite start temperature (As), and finishes at the austenite finish temperature (Af).

In some embodiments, heating the distal portion 520 of the guidewire 500 can be affected by running current through the guidewire 500 via the user interface 420 at handle 400 **(****FIG. 4A****).** The resistivity of the material forming the distal portion 520 creates heating which can be controlled with electrical power modulation in the user interface 420. In some embodiments, the Af temperature of the first segment 522 can be selected to be slightly above body temperature so that the actuation of the distal portion 520 of the guidewire 500 does not require heating above a safe limit for blood contact. In such cases, the distal portion 520 of the guidewire 500 will not deflect during storage or advancing of the guidewire until a current is applied to the guidewire 500 and the Af temperature of the first segment 522 is reached to be slightly raised above body temperature.

It should be noted that having the first segment 522 and second segment 524 of the distal portion 520 coupled in a laminated configuration allows the distal portion 520 of the guidewire 500 to achieve larger deflections with a substantially short first segment 522. The amount of deflection of the distal portion 520 of the guidewire 500 is governed by the differential properties of the first segment 522 and second segment 524, and/or an amount of current being delivered to the distal portion 520 of the guidewire 500. In some embodiments, the non-shape-memory second segment 524 may have mechanical properties independently tuned and/or selected to also provide shapeability and functionality of the distal portion 520 of the guidewire 500.

In some embodiments, the first segment 522 of the distal portion 520 of the guidewire 500 may have a length that is anywhere between 5 mm to 15 mm, or that is anywhere between 5 mm to 20 mm, or that is anywhere between 5 mm to 3,400 mm (e.g., approximately, the full length the guidewire). The second segment 524 may be shorter than the first segment 522, the same length as the first segment 522, or longer than the first segment 522.

The deflection of the distal portion 520 of the guidewire 500 is produced due to the magnitude of differences between the coefficient of thermal actuation of the first segment 522 and the non-thermal actuation of the second segment 524. In some embodiments, the thermal/electrical actuation of the distal portion 520 of the guidewire 500 creates a four percent or greater (≥ 4%) shorten/contraction or length change differential over a relatively small change in temperature range (e.g., 5° Fahrenheit (2.77°C) or higher, 10° Fahrenheit (5.55°C) or higher, 15° Fahrenheit (8.33°C) or higher, etc.), such that large distal portion 520 deflection may be achieved with small temperature changes. In some cases, the tightest achievable radius of curvature for the distal portion 520 of the guidewire 500 is in the range of 0.05" (e.g. 0.05" +/- 0.02") (1.27mm +/- 0.508mm). In other cases, the radius of curvature for the distal portion 520 of the guidewire 500 may be higher, such as 0.1", 0.2", 0.4", 0.6", etc., +/- 0.05") (0.254mm, 0.508mm, 1.016mm, 1.524mm, etc,, +/- 1.27mm).

In some embodiments, the deflection angle of the distal portion 520 of the guidewire 500 may be controlled by thermal/current modulation applied to the guidewire 500. The current may be applied using a monopolar technique, where the current passes from the guidewire 500, through the patient's tissue to a return pad to complete the electric current circuit, or using a bipolar technique, where the current return path is along the guidewire 500 back to the electrical controller at the user interface 420. Also, in some embodiments, the curvature of the bending of the distal portion 520 may be selectively adjusted using the control at the handle 400. The control may be manipulated to change an amount of current applied to the distal portion 520 of the guidewire 500. In one mode of operation, the amount of current may be increased to increase a curvature of the bending at the distal portion 520 of the guidewire 500. In another mode of operation, the amount of current may be decreased in decrease a curvature of the bending at the distal portion 520 of the guidewire 500.

Furthermore, in some embodiments, annealing parameters may be tuned to achieve a desired level of elasticity and shapeability in the material (e.g., Nitinol) forming the distal portion 520 of the guidewire 500. In some embodiments, the second segment 524 of the distal portion 520 of the guidewire 500 may be heat treated to a semi or partially annealed condition, such that the second segment 524 may be shapeable and could retain a standard-like shapeable tip guidewire, yet the distal portion 520 of the guidewire 500 still includes the thermo-electrically actuated first segment 522 to actively deflect the distal portion 520 when needed. In such cases, active deflection (e.g., applying current or heat to the guidewire 500) may not be required for one usage of the guidewire 500, but the active deflection may be used when navigating the guidewire 500 through more challenging and tortuous vasculature in another usage.

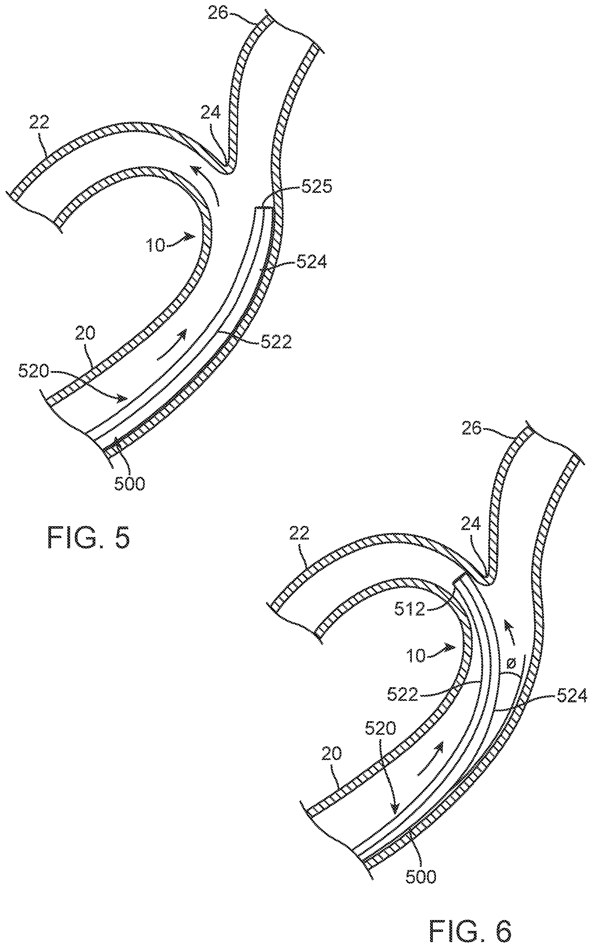

**FIGS. 5-6** illustrate a method, not forming part of the present invention, of accessing a bifurcated vasculature 10 using the guidewire 500. The bifurcated vasculature 10 includes a main blood vessel 20, a first blood vessel branch 22, a second blood vessel branch 26, and a bifurcated angle 24 between the first 22 and second 26 branches. The guidewire 500 having the distal portion 520 is advanced through the main blood vessel 20 and maneuvered to access a target site within the first blood vessel branch 22.

As the guidewire 500 is being advanced inside the patient, the user interface 420 may be operated by the user to actuate the guidewire 500 to bend in a desired manner. With the assistance of known imaging technologies and the marker 525 disposed at the distal end 512 of the guidewire 500, the user can determine the location of the distal portion 520 of the guidewire 500 within the main blood vessel 20 **(****FIG. 5****).** If the user determines that bending of the distal portion 520 is desired, the user may operate the user interface 420 to apply heat or current to the distal portion 520 of the guidewire 500 to thermally or electrically actuate the first segment 522 composed of shape-memory material, such that the distal portion 520 of the guidewire 500 deflects towards the desired first blood vessel branch 22 **(****FIG. 6****).** The distal portion 520 of the guidewire 500 may then be advanced distally into the first blood vessel branch 22. To achieve this deflection for distal portion 520 of the guidewire 500, the angle of distal portion 520 relative to the longitudinal axis of elongate body of the guidewire 500, as illustrated by angle " Φ", can range from about five degrees to about 60 degrees, or more.

As illustrated above, the bending of the distal portion 520 allows the distal end 512 of the guidewire 500 to be steered through different curvatures along a passage way (e.g., blood vessel) inside the patient. In some embodiments, the guidewire body 515 may be rotated about its longitudinal axis to allow the bending to occur at different bending planes. Also, in some embodiments, a degree (e.g., curvature, angle, etc.) of bending of the guidewire 500 may be adjusted by varying a magnitude of the heat or current provided by the user interface 420.

After the distal end 512 of the guidewire 500 has been desirably positioned inside the patient, the elongated member 120 and/or sheath 110 of **FIG. 3** may then be advanced over the guidewire 500, and may be utilized in a medical procedure to diagnose and/or treat the patient. For example, the elongated member 120 and/or the sheath 110 may be used to deliver a substance (e.g., drug, medicine, contrast, saline, etc.), deploy a device (e.g., implant, tissue dissector, imaging scope, treatment energy source, etc.), or perform other functions in different embodiments.

It should be noted that the guidewire 500 is not limited to the examples of **FIGS. 4A-4B****,** and that the guidewire 500 may have other configurations in other embodiments. In other embodiments, the guidewire 500 may include more than two distal segments that are coupled to form the distal portion 520 of the guidewire 500. For example, in other embodiments, the distal portion 520 may have three distal segments that are stacked and coupled together to form the distal portion 520 of the guidewire 500. In other embodiments, the guidewire 500 may include other components along a longitudinal axis of the guidewire 500, such as an outer jacket, sleeve, reinforcement segments, coils, radiopaque coatings, markers, or the like.

In accordance with the invention, instead of being a guidewire, the wire 500 may be a pushwire, or a delivery wire.

In some embodiments, the wire 500 may have a stiffer proximal portion compared to the distal portion 520. The stiffer proximal portion may be at least 30%, at least 50%, at least 70%, or at least 80%, of an entire length of the wire 500. Also, in some embodiments, the wire 500 may have a proximal portion that is proximal to the distal portion 520, wherein the proximal portion may be made from stainless steel, Nitinol, Cobalt-Chromium alloy (e.g., MP35N alloy), other alloys, or combination thereof.

In other embodiments, the wire 500 may be formed of stainless steel (or other rigid alloy) along a proximal portion, and having nitinol at the distal portion 520.

In other embodiments, the wire 500 may include a hybrid core. For example, a portion of the body 515 that is proximal to the distal portion 520 may be made from Nitinol and another material (e.g., stainless steel) to provide a stiffer proximal portion for the wire 500.

In further embodiments, the wire 500 may have a Nitinol segment that extends the full length of the wire 500. In such cases, one or more portions of the Nitinol core may be heat treated to provide shape-memory characteristics, as similarly described herein.

In further embodiments, the wire 500 may function as a core wire or backbone for a variety of elongated medical devices, such as complex guidewires, sheath, catheters, or the like. The compact size of the wire 500 allows incorporation of it as a core wire into sheath, catheters or their like without impacting other performance characteristics (torque transmission, stiffness, tip shapeability, etc.).

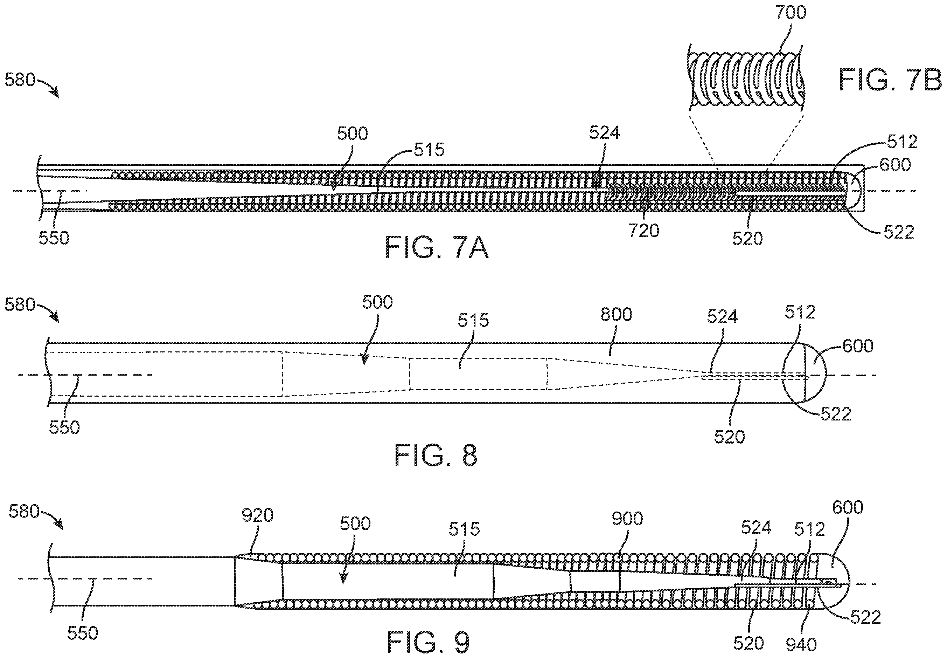

**FIGS. 7A-9** illustrate different examples of medical devices that incorporate the wire 500 described herein. The wire 500 in **FIGS. 7A-9** include a reduced diameter (i.e., taper) from the proximal end (not shown) and/or the body 515 towards the distal end 512 of the wire 500. The tapering of the wire 500 may be a constant reduction of diameter of the wire 500 along a longitudinal axis 550 (as shown in **FIG. 7A****),** or may have distinct transitions between tapered sections (as shown in **FIGS. 8-9****),** or a combination thereof.

As shown in **FIG. 7A****,** the medical device 580 includes the wire 500 and an outer jacket 700 disposed around at least a portion of the distal portion 520 of the wire 500. The outer jacket 700 may be any tubular member, and may be made from any suitable materials, such as metal, polymer, etc. In some embodiments, the outer jacket 700 may be made from nitinol. As better appreciated in detailed **FIG. 7B****,** the outer jacket 700 includes a plurality of slots and/or openings to increase flexibility. By way of nonlimiting examples, the outer jacket 700 may be implemented using slotted hypotube, coiled sleeve, tungsten-loaded polymer sleeve, or a combination thereof. The medical device 580 may further include a coil 720 disposed around the distal portion 520 of the wire 500 and concentrically disposed between the distal portion 520 of the wire 500 and the outer jacket 700. The coil 720 may be made of radiopaque material and/or platinum tungsten. The medical device 580 further includes a blunt atraumatic tip 600 coupled to the distal portion 520 of the wire 500.

**FIG. 8** illustrates another medical device 580 having the wire 500 and an outer jacket 800 concentrically disposed around at least the distal portion 520 of the wire 500. The outer jacket 800 is composed of suitable polymeric material. In other embodiments, the outer jacket 800 may be made from other materials. The medical device 580 further includes a blunt atraumatic tip 600 coupled to the distal portion 520 of the wire 500.

**FIG. 9** illustrates another medical device 580 having the wire 500 and a coil 900 concentrically disposed around at least the distal portion 520 of the wire 500. As shown in the figure, a proximal end 920 of the coil 900 is secured to the body 515 of the wire 500, while the distal end 940 of the coil 900 is secured to the distal portion 520 of the wire 500. The securing may be accomplished using an adhesive, welding, mechanical connector, fusion, etc. The medical device 580 further includes a blunt atraumatic tip 600 coupled to the distal portion 520 of the wire 500.

Parts of the description and drawings referring to embodiments which are not covered by the claims are not presented as embodiments of claimed subject matter but as background examples useful for understanding the invention and may, if applicable, provide details to further specify embodiments claimed or described in this application.

## Claims

1. A medical device (580), comprising:
an elongated member (500) having a proximal end (510), a distal end (512), and a body (515) extending between the proximal end (510) and the distal end (512);
wherein at least a first portion of the elongated member (500) comprises a first segment (522) made from a shape-memory material, and a second segment (524) made from a non-shape-memory material, the first portion being a distal portion (520) of the elongated member; and
wherein the first segment (522) is configured to undergo length change to cause the distal portion of the elongated member (500) to bend in response to an application of heat or current;
wherein the elongated member (500) is a guidewire, a pushwire, a delivery wire, or a wire of the medical device;
**characterized in that**:
the first segment (522) and the second segment (524) of the distal portion (520) of the elongated member (500) are fixedly attached to each other at one or more points along a longitudinal axis of the elongated member (500) via an adhesive, solder, or weld along their respective longitudinal sides to form a laminated configuration.

2. The medical device (580) of claim 1, wherein the first segment (522) is configured to change length in response to an increase in temperature of the first segment (522) that is above a body temperature.

3. The medical device (580) of claim 2, wherein the temperature is at least ten degrees Fahrenheit (5.55 °C) above the body temperature.

4. The medical device (580) of any of claims 1-3, wherein the shape-memory material of the first segment (522) comprises shape-memory Nitinol, and the non-shape-memory material of the second segment (524) comprises non-shape-memory Nitinol.

5. The medical device (580) of any of claims 1-3, wherein the elongated member (500) comprises a second portion proximal to the distal portion (520), and wherein the second portion is made out of a different material than the distal portion.

6. The medical device (580) of claim 1, wherein the elongated member (500) comprises a second portion proximal to the distal portion (520), and wherein the second portion is made out of stainless steel or non-shape memory Nitinol, and the distal portion (520) is made out of shape-memory Nitinol.

7. The medical device (580) of any of claims 1-6, further comprising a jacket (700) or a slotted tube disposed around at least the distal portion (520) of the elongated member (500).

8. The medical device (580) of any of claims 1-7, further comprising a marker (525) coupled to the distal portion (520) of the elongated member (500).

9. The medical device (580) of claim 1, wherein the first segment (522) is configured to undergo the length change in response to a temperature that is above a body temperature, and wherein the second segment (524) is configured to undergo zero length change or less length change compared to the first segment (522) in response to the temperature.

10. The medical device (580) of claim 5, wherein the second portion comprises stainless steel, and the distal portion (520) comprises Nitinol.

11. The medical device (580) of claim 5, wherein the second portion of the elongated member (500) comprises non-shape-memory Nitinol, and the first segment (522) of the distal portion (520) of the elongated member (500) comprises shape-memory Nitinol.

12. A medical treatment system including the medical device (580) of claim 1, the system further comprising an energy source coupled to the elongated member (500), wherein the energy source is configured to deliver a current to the elongated member (500) to increase a temperature of the distal portion (520) of the elongated member (500).

13. The medical treatment system of claim 12, wherein the second segment (524) is longer or shorter than the first segment (522).

14. The medical device (580) of claim 1, wherein the medical device (580) is a guidewire.

15. The medical device (580) of claim 1, wherein the medical device (580) is a delivery system.

## Patentansprüche

1. Medizinische Vorrichtung (580), umfassend:
ein längliches Element (500), das ein proximales Ende (510), ein distales Ende (512) und einen Körper (515), der sich zwischen dem proximalen Ende (510) und dem distalen Ende (512) erstreckt, aufweist;
wobei mindestens ein erster Abschnitt des länglichen Elements (500) ein erstes Segment (522), das aus einem Formgedächtnismaterial hergestellt ist, und ein zweites Segment (524), das aus einem Nicht-Formgedächtnismaterial hergestellt ist, umfasst, wobei der erste Abschnitt ein distaler Abschnitt (520) des länglichen Elements ist; und
wobei das erste Segment (522) so ausgestaltet ist, dass es als Reaktion auf eine Anwendung von Wärme oder Strom eine Längenänderung erfährt, die bewirkt, dass sich der distale Abschnitt des länglichen Elements (500) biegt;
wobei das längliche Element (500) ein Führungsdraht, ein Schiebedraht, ein Zuführdraht oder ein Draht der medizinischen Vorrichtung ist;
**dadurch gekennzeichnet, dass**:
das erste Segment (522) und das zweite Segment (524) des distalen Abschnitts (520) des länglichen Elements (500) so an einem oder an mehreren Punkten entlang einer Längsachse des länglichen Elements (500) mittels Klebstoff, Lötmittel oder Schweißung entlang ihrer jeweiligen Längsseiten fest aneinander befestigt sind, dass sie eine laminierte Ausgestaltung bilden.

2. Medizinische Vorrichtung (580) nach Anspruch 1, wobei das erste Segment (522) so ausgestaltet ist, dass es seine Länge als Reaktion auf einen Temperaturanstieg des ersten Segments (522), der über einer Körpertemperatur liegt, ändert.

3. Medizinische Vorrichtung (580) nach Anspruch 2, wobei die Temperatur mindestens zehn Grad Fahrenheit (5,55 °C) über der Körpertemperatur liegt.

4. Medizinische Vorrichtung (580) nach einem beliebigen der Ansprüche 1-3, wobei das Formgedächtnismaterial des ersten Segments (522) Nitinol mit Formgedächtnis umfasst und das Nicht-Formgedächtnismaterial des zweiten Segments (524) Nitinol ohne Formgedächtnis umfasst.

5. Medizinische Vorrichtung (580) nach einem beliebigen der Ansprüche 1-3, wobei das längliche Element (500) einen zweiten Abschnitt proximal zu dem distalen Abschnitt (520) umfasst, und wobei der zweite Abschnitt aus einem anderen Material als der distale Abschnitt hergestellt ist.

6. Medizinische Vorrichtung (580) nach Anspruch 1, wobei das längliche Element (500) einen zweiten Abschnitt proximal zu dem distalen Abschnitt (520) umfasst, und wobei der zweite Abschnitt aus rostfreiem Stahl oder Nitinol ohne Formgedächtnis hergestellt ist, und der distale Abschnitt (520) aus Nitinol mit Formgedächtnis hergestellt ist.

7. Medizinische Vorrichtung (580) nach einem beliebigen der Ansprüche 1-6, die ferner einen Mantel (700) oder ein geschlitztes Rohr umfasst, der bzw. das um mindestens den distalen Abschnitt (520) des länglichen Elements (500) herum angeordnet ist.

8. Medizinische Vorrichtung (580) nach einem beliebigen der Ansprüche 1-7, die ferner eine Markierung (525) umfasst, die mit dem distalen Abschnitt (520) des länglichen Elements (500) verbunden ist.

9. Medizinische Vorrichtung (580) nach Anspruch 1, wobei das erste Segment (522) so ausgestaltet ist, dass es die Längenänderung als Reaktion auf eine Temperatur erfährt, die über einer Körpertemperatur liegt, und wobei das zweite Segment (524) so ausgestaltet ist, dass es als Reaktion auf die Temperatur keine Längenänderung oder eine im Vergleich zu dem ersten Segment (522) geringere Längenänderung erfährt.

10. Medizinische Vorrichtung (580) nach Anspruch 5, wobei der zweite Abschnitt rostfreien Stahl umfasst, und der distale Abschnitt (520) Nitinol umfasst.

11. Medizinische Vorrichtung (580) nach Anspruch 5, wobei der zweite Abschnitt des länglichen Elements (500) Nitinol ohne Formgedächtnis umfasst, und das erste Segment (522) des distalen Abschnitts (520) des länglichen Elements (500) Nitinol mit Formgedächtnis umfasst.

12. Medizinisches Behandlungssystem, das die medizinische Vorrichtung (580) nach Anspruch 1 umfasst, wobei das System ferner eine Energiequelle umfasst, die mit dem länglichen Element (500) verbunden ist, wobei die Energiequelle so ausgestaltet ist, dass sie dem länglichen Element (500) einen Strom zuführt, um eine Temperatur des distalen Abschnitts (520) des länglichen Elements (500) zu erhöhen.

13. Medizinisches Behandlungssystem nach Anspruch 12, wobei das zweite Segment (524) länger oder kürzer als das erste Segment (522) ist.

14. Medizinische Vorrichtung (580) nach Anspruch 1, wobei die medizinische Vorrichtung (580) ein Führungsdraht ist.

15. Medizinische Vorrichtung (580) nach Anspruch 1, wobei die medizinische Vorrichtung (580) ein Zuführsystem ist.

## Revendications

1. Dispositif médical (580), comprenant :
un élément allongé (500) présentant une extrémité proximale (510), une extrémité distale (512), et un corps (515) s'étendant entre l'extrémité proximale (510) et l'extrémité distale (512) ;
dans lequel au moins une première partie de l'élément allongé (500) comprend un premier segment (522) constitué d'un matériau à mémoire de forme, et un deuxième segment (524) constitué d'un matériau sans mémoire de forme, la première partie étant une partie distale (520) de l'élément allongé ; et
dans lequel le premier segment (522) est configuré pour subir un changement de longueur pour amener la partie distale de l'élément allongé (500) à se courber en réponse à une application de chaleur ou de courant ;
dans lequel l'élément allongé (500) est un fil de guidage, un fil de poussée, un fil de distribution, ou un fil du dispositif médical ;
**caractérisé en ce que** :
le premier segment (522) et le deuxième segment (524) de la partie distale (520) de l'élément allongé (500) sont reliés fixement l'un à l'autre en un ou plusieurs points le long d'un axe longitudinal de l'élément allongé (500) par le biais d'un adhésif, d'un brasage, ou d'une soudure le long de leurs côtés longitudinaux respectifs pour former une configuration stratifiée.

2. Dispositif médical (580) selon la revendication 1, dans lequel le premier segment (522) est configuré pour changer de longueur en réponse à une augmentation dans la température du premier segment (522), laquelle est supérieure à une température corporelle.

3. Dispositif médical (580) selon la revendication 2, dans lequel la température est supérieur d'au moins dix degrés Fahrenheit (5,55 °C) à la température corporelle.

4. Dispositif médical (580) selon l'une quelconque des revendications 1 à 3, dans lequel le matériau à mémoire de forme du premier segment (522) comprend du nitinol à mémoire de forme, et le matériau sans mémoire de forme du deuxième segment (524) comprend du nitinol sans mémoire de forme.

5. Dispositif médical (580) selon l'une quelconque des revendications 1 à 3, dans lequel l'élément allongé (500) comprend une deuxième partie proximale à la partie distale (520), et dans lequel la deuxième partie est composée d'un matériau différent de celui de la partie distale.

6. Dispositif médical (580) selon la revendication 1, dans lequel l'élément allongé (500) comprend une deuxième partie proximale à la partie distale (520), et dans lequel la deuxième partie est composée d'acier inoxydable ou de nitinol sans mémoire de forme, et la partie distale (520) est composée de nitinol à mémoire de forme.

7. Dispositif médical (580) selon l'une quelconque des revendications 1 à 6, comprenant en outre une gaine (700) ou un tube fendu disposé(e) au moins autour de la partie distale (520) de l'élément allongé (500).

8. Dispositif médical (580) selon l'une quelconque des revendications 1 à 7, comprenant en outre un marqueur (525) accouplé à la partie distale (520) de l'élément allongé (500).

9. Dispositif médical (580) selon la revendication 1, dans lequel le premier segment (522) est configuré pour subir le changement de longueur en réponse à une température supérieure à une température corporelle, et dans lequel le deuxième segment (524) est configuré pour subir un changement de longueur nul ou un changement de longueur inférieur en comparaison avec le premier segment (522) en réponse à la température.

10. Dispositif médical (580) selon la revendication 5, dans lequel la deuxième partie comprend de l'acier inoxydable, et la partie distale (520) comprend du nitinol.

11. Dispositif médical (580) selon la revendication 5, dans lequel la deuxième partie de l'élément allongé (500) comprend du nitinol sans mémoire de forme, et le premier segment (522) de la partie distale (520) de l'élément allongé (500) comprend du nitinol à mémoire de forme.

12. Système de traitement médical incluant le dispositif médical (580) selon la revendication 1, le système comprenant en outre une source d'énergie accouplée à l'élément allongé (500), dans lequel la source d'énergie est configurée pour distribuer un courant à l'élément allongé (500) pour augmenter une température de la partie distale (520) de l'élément allongé (500).

13. Système de traitement médical selon la revendication 12, dans lequel le deuxième segment (524) est plus long ou plus court que le premier segment (522).

14. Dispositif médical (580) selon la revendication 1, dans lequel le dispositif médical (580) est un fil de guidage.

15. Dispositif médical (580) selon la revendication 1, dans lequel le dispositif médical (580) est un système de distribution.
